# EUROPÄISCHE PATENTSCHRIFT

(11) **EP 2 396 582 B1**
(45) Veröffentlichungstag und Bekanntmachung des Hinweises auf die Patenterteilung: **18.07.2018**
(21) Anmeldenummer: 10702626.2
(22) Anmeldetag: 03.02.2010
(51) Int. Cl.: F16L 11/118, F16L 11/20

(54) **VERFAHREN ZUR HERSTELLUNG VON BESTRAHLUNGSMODULEN**
METHOD FOR PRODUCING RADIATION MODULES
PROCÉDÉ DE FABRICATION DE MODULES D'IRRADIATION

(30) Priorität: 16.02.2009 DE 102009009108
(43) Veröffentlichungstag der Anmeldung: 21.12.2011
(73) Patentinhaber: Bayer Aktiengesellschaft, 51373 Leverkusen (DE)
(72) Erfinder: POGGEL, Martin, 50668 Köln (DE); KAULING, Jörg, 51427 Bergisch Gladbach (DE); SCHMIDT, Sebastian, 42781 Haan (DE); BECKERS, Erhard, 51399 Burscheid (DE)
(74) Vertreter: BIP Patents
(86) Internationale Anmeldenummer: PCT/EP2010/000648
(87) Internationale Veröffentlichungsnummer: WO 2010/091815

(56) Entgegenhaltungen:
- EP-A1- 1 464 342
- DE-A1- 2 061 299
- US-B2- 6 446 661

## Beschreibung

Gegenstand der vorliegenden Erfindung ist eine Vorrichtung und ein Verfahren zum Aufbringen flexibler, profilierter Hohlzylinder auf zylinderförmige Körper. Profilierte Hohlzylinder, die auf einen zylinderförmigen Körper aufgebracht sind, bilden Module, die zur Bestrahlung von fluiden Medien mit elektromagnetischer Strahlung geeignet sind. Gegenstand der vorliegenden Erfindung ist daher auch ein Verfahren zur Herstellung von Bestrahlungsmodulen sowie mittels des erfindungsgemäßen Verfahrens hergestellte Bestrahlungsmodule.

Ein Zylinder ist ein Körper, der von zwei parallelen, ebenen Flächen (Grund- und Deckfläche) und einer Mantelfläche, die von parallelen Geraden gebildet wird, begrenzt wird.
Er entsteht durch Verschiebung einer ebenen Fläche oder Kurve entlang einer Geraden, die nicht in dieser Ebene liegt. Eine spezielle Ausführungform eines Zylinders ist ein Kreiszylinder. Er entsteht durch Verschiebung eines Kreises parallel zu einer Geraden durch den Kreismittelpunkt, wobei die Gerade nicht in der Kreisebene liegt. Ein Kreiszylinder wird begrenzt von zwei parallelen Kreisflächen (Grundfläche und Deckfläche) und der so genannten Mantelfläche.

Unter einem Hohlzylinder wird hier und im Folgenden eine spezielle Ausführungsform eines Zylinders verstanden, die dadurch gekennzeichnet ist, dass von der Grundfläche ein Kanal parallel zur Mantelfläche bis zur Deckfläche verläuft. Ein Rohr ist ein Beispiel eines Hohlzylinders.
Unter einem flexiblen Hohlzylinder wird ein Hohlzylinder verstanden, der zu einem gewissen Grad gebogen und/oder gestreckt werden kann. Der Grad der Streckung und/oder Biegung beträgt dabei mindestens 1% der Länge und/oder des Durchmessers des Hohlzylinders. Ein Beispiel eines flexiblen Hohlzylinders ist ein Schlauch.
Unter einem profilierten Hohlzylinder wird ein Hohlzylinder verstanden, der entlang seiner Mantelfläche ein Profil aufweist. Dieses Profil kann z.B. wellenförmig oder wendelförmig sein. Beispiele für profilierte Hohlzylinder sind Wellschläuche und Wellrohre sowie Wendelschläuche und Wendelrohre.
Ein flexibler profilierter Hohlzylinder ist dadurch gekennzeichnet, dass er zu einem gewissen Grad (mindestens 1% der Länge und/oder des Durchmessers des Hohlzylinders, bevorzugt 5 bis 20%) entlang seiner Längsachse gestreckt werden kann.

Flexible, profilierte Hohlzylinder spielen in vielen Bereichen der Technik eine große Rolle. In der Automobilindustrie, dem Maschinen- und Anlagenbau sowie der Medizintechnik werden Wellschläuche zum Schutz und zur Bündelung elektrischer oder anderer Leitungen und auch zur Ausführung flexibler Verbindung zu peripheren Geräten benutzt. Bei der Elektroinstallation werden Plaste-Wellschläuche vorwiegend als bzw. anstelle so genannter Leerrohre sowohl im Außenbereich, vor allem aber innerhalb von Gebäude-Wänden und -decken, eingesetzt. Wellrohre werden als Wärmeübertrager (z.B. aus Edelstahl in Pufferspeichern oder aus Kunststoff für Fußbodenheizungen) eingesetzt. Die gewellte Struktur dient dabei zur Vergrößerung der Oberfläche für einen möglichst guten Wärmetransport. Wellrohr-Stücke (Metallbälge) werden zum Ausgleich von Achsversätzen oder zur Kompensation von Längen- und Winkeländerungen eingesetzt.

In den Anmeldungen WO-A 02/38502, WO-A 02/38191, WO-A 07/096057 und EP-A 1464342 wird die Verwendung von Wendelschläuchen in Vorrichtungen zur Bestrahlung von flüssigen Medien zum Zwecke der Sterilisation beschrieben.

In vielen der oben genannten Anwendungen und Verwendungen, insbesondere in der Verwendung profilierter Hohlzylinder in Bestrahlungsvorrichtungen werden die profilierten Holzylinder auf einen zylinderförmigen Körper aufgebracht. In WO07/096057A2 ist beispielsweise ein so genanntes Bestrahlungsmodul beschrieben, das dadurch gekennzeichnet ist, dass über ein inneres Stützrohr ein Wendelschlauch formschlüssig aufgebracht ist. Dadurch entsteht zwischen dem Stützrohr und dem Wendelschlauch ein Kanal, der helikal von einem Ende des Wendelschlauches um das Stützrohr zum anderen Ende des Wendelschlauches verläuft. Eine solche Vorrichtung ist sehr gut zur Bestrahlung von fluiden Medien, die den Kanal durchströmen, geeignet. Hierzu wird in das Stützrohr und/oder um den Wendelschlauch eine oder mehrere Strahlungsquellen angeordnet, welche das in dem Kanal strömende Medium bevorzugt mit UV-Strahlung, besonders bevorzugt mit UV-C-Strahlung bestrahlen. Die Bestrahlung führt zur Reduktion von Mikroorganismen und/oder Viren oder zur chemischen Umsetzung in einem photochemischen Reaktor
Das besondere Kennzeichen des durchströmten Kanals ist eine intensive, über die gesamte Länge herrschende gleichförmige Quervermischung senkrecht zur Hauptrichtung der Produktströmung sowie eine durch turbulente Produktströmung eingeengte Verweilzeitverteilung. Durch die Quervermischung wird gewährleistet, dass die von der Strahlenquelle entfernteren Fluidschichten, die besonders bei stark lichtabsorbierenden Medien keine oder wenig UV-Strahlung erhalten, einen intensiven Austausch mit den UV-bestrahlten Schichten nahe der Strahlenquelle eingehen. Dies und die enge Verweilzeitverteilung führt dazu, dass alle Fluidelemente eine gleichmäßige und einheitliche Bestrahlungsdauer und -intensität erfahren, die sich durch die Strömungsgeschwindigkeit und die Intensität der Strahlungsquelle auf die jeweiligen Bedürfinsse anpassen lassen.
So kann gewährleistet werden, dass eine effektive Reduktion an Mikroorganismen und/oder Viren in dem Medium erfolgt. Bei Medien, bei denen eine zu starke Bestrahlung zu Schäden führen kann, wird die Gefahr, dass es durch eine ungünstig breite Verweilzeitverteilung zu einer zu starken Strahlenbelastung und damit teilweisen Schädigung kommt, wirksam vermindert.

Dafür ist es jedoch erforderlich, dass der Wendelschlauch das Stützrohr formschlüssig umschließt und so nur ein einziger Kanal gebildet wird, der helikal um das Stützrohr verläuft. Querströmungen zwischen benachbarten Kanalwindungen sind zu vermeiden, da diese zu einer Verbreiterung der Verweilzeitverteilung führen würden.

Die Herstellung des Kanals erfolgt in EP-A 1464342 durch Aufziehen eines gewendelten Schlauches auf einen zylindrischen Stützkörper. Durch eine geeignete Geometrie des gegenüber dem Stützkörper im Innendurchmesser geringfügig verminderten Wendelschlauches wird eine straffe, kraftschlüssige Verbindung zwischen beiden Elementen hergestellt. Hierdurch lassen sich die sonst durch Spalte zwischen den Kanalwindungen verursachten axialen Kurzschlussströmungen und die damit einhergehende Verbreiterung der Verweilzeitverteilung verhindern.

Das Aufbringen eines flexiblen, profilierten Hohlzylinders auf einen zylinderförmigen Körper stellt jedoch ein technisches Problem dar, insbesondere wenn der kleinste Innendurchmesser des profilierten Hohlzylinders kleiner als oder genauso groß wie der Außendurchmesser des zylinderförmigen Körpers ist.
Ein profilierter Hohlzylinder weist aufgrund seiner Profilierung der Mantelfläche eine Reihe von Engstellen (hier auch als Windungen bezeichnet) auf, die beim Aufbringen auf einen zylinderförmigen Körper überwunden werden müssen. Das Aufbringen erfolgt in der Regel derart, dass der zylinderförmige Körper in den Kanal des profilierten Hohlzylinders eingeschoben und/oder der profilierte Hohlzylinder auf den zylinderförmigen Körper aufgezogen wird. Dabei erhöht sich bei jeder Windung des Hohlzylinders, die bereits auf den Körper aufgebracht worden ist, die Reibung zwischen Hohlzylinder und zylinderförmigem Körper, so dass die Kraft, die auf den Hohlzylinder und/oder auf den Körper ausgeübt werden muss, mit der Länge des Hohlzylinders wächst.
Je nach verwendeten Materialien von Hohlzylinder und zylinderförmigem Körper kann es dabei zu Abnutzungen, Rissen, Kratzern bis hin zum Zerbrechen der Komponenten führen.
Ferner führt das in EP-A 1464342 zur Herstellung einer Bestrahlungsvorrichtung beschriebene "Aufziehen" eines gewendelten Schlauches auf einen zylindrischen Stützkörper zu nicht definierten Kanalgeometrien. Durch das Aufziehen und die dabei entstehenden Spannungen kann die Wendelgeometrie beeinflusst werden. Einzelne Windungen können gequetscht werden. Engstellen in einzelnen Kanalwindungen führen zu Druckverlusten. Durch Quetschungen einzelner Kanäle lassen sich Spalte zwischen benachbarten Kanalwindungen kaum verhindern. Das Ergebnis ist eine nicht gut definierte und nicht reproduzierbare Bestrahlungsvorrichtung. Zusätzlich kann es durch zu starke Reibung auch zu Partikelabrieb kommen. Diese Partikel sind für diverse Anwendungen kritisch, wie beispielsweise im pharmzeutischen Umfeld.

Es besteht somit Bedarf nach einer Methode zum Aufbringen von flexiblen, profilierten Hohlzylindern auf zylinderförmige Körper, die einfach auszuführen ist und die mechanische Belastung der Komponenten derart niedrig hält, dass eine Abnutzung und/oder gar Zerstörung der Komponenten ausgeschlossen werden kann.

Weiterhin besteht Bedarf an einem Verfahren zum Aufbringen von flexiblen, profilierten Hohlzylindern auf zylinderförmige Körper, das im industriellen Maßstab durchgeführt werden kann. Insbesondere die zumindest teilweise Automatisierung des Prozesses ist hier von enormer Wichtigkeit, um z.B. die in den Anmeldungen WO-A 02/38502, WO-A 02/38191, WO-A 07/096057 und EP-A 1464342 beschriebenen Bestrahlungsvorrichtungen in einem industriellen Maßstab kostengünstig und reproduzierbar herstellen zu können.

Ausgehend vom bekannten Stand der Technik stellt sich demnach die Aufgabe, ein Verfahren bereitzustellen, mit dem flexible, profilierte Hohlzylinder auf zylinderförmige Körper aufgebracht werden können, ohne dass eine Komponente hierbei einen Schaden nimmt. Das gesuchte Verfahren soll zumindest teilweise automatisierbar sein. Es soll einfach und kostengünstig auszuüben sein. Ferner stellt sich die Aufgabe, eine Vorrichtung bereitszustellen, mit dem das gesuchte Verfahren ausgeübt werden kann. Die gesuchte Vorrichtung soll intuitiv zu bedienen und ebenfalls kostengünstig sein.
Das gesuchte Verfahren soll die repoduzierbare Herstellung von Bestrahlungsvorrichtungen ermöglichen, die über wohldefinierte Kanalgeometrien verfügen und die damit wirkungsvolle und reproduzierbare Ergebnisse bei der Bestrahlung von fluiden Medien mittels elektromagnetischer Strahlung, zum Beispiel lassen sich Spalte zwischen benachbarten Kanalwindungen kaum verhindern. Das Ergebnis ist eine nicht gut definierte und nicht reproduzierbare Bestrahlungsvorrichtung. Zusätzlich kann es durch zu starke Reibung auch zu Partikelabrieb kommen. Diese Partikel sind für diverse Anwendungen kritisch, wie beispielsweise im pharmzeutischen Umfeld.

DE-A 2061299 betrifft ein biegsames gewelltes Fadenwickelrohr. Das biegsame Fadenwickelrohr kann insbesondere als Rohr- oder Schlauchverbindungsstück verwendet werden und es ist so ausgebildet, dass es eine gut abdichtende Verbindung zwischen zwei Rohren o.dgl. herbeiführt.

Das Rohr ist biegsam und kann leicht angebracht und wieder gelöst werden, weißt aber trotzdem eine hinreichende Festigkeit auf, um hohen Innendrücken, Aussendrücken und Längekräften zu widerstehen. Das Rohr oder der Schlauch ist gewellt, so dass Biegsamkeit und gute Festigkeit gewährleistet sind. Weiterhin gibt die Wellung dem Rohr versteifende Rippen, so dass die Bohrung oder der Innenraum des Rohrs hinreichend offen bleibt, wenn das Rohr über seine Länge gebogen oder gekrümmt wird.

EP-A 1464342 betrifft wiederum eine Technik zur sicheren und produktschonenden UV-Bestrahlung und Hitze-Sterilisierung flüssiger Medien und insbesondere Mikroorganismen und/oder Viren enthaltender Flüssigkeiten, wie bspw. Nahrungsmittel, Milch- oder Fruchtsaftprodukte, chemische oder pharmazeutische Produkten, Virus-Vakzine, gentechnisch erzeugte Wirkstoffe oder Proteine, Wirkstoffe oder Proteine aus transgenen Tieren oder Pflanzen und Blutplasma oder aus Blutplasma gewonnene Produkte.

Ein gemeinsames Merkmal der Bestrahlung mit UV-Licht und der Behandlung mit Hitze ist eine die Inaktivierungsreaktion begleitende unerwünschte Produktschädigung, deren Ausmaß durch geeignete reaktionstechnische und konstruktive Maßnahmen minimiert werden muss. In EP-A 1464342 ist ein kontinuierliches Verfahren zur Sterilisation und gegebenenfalls Virusinaktivierung fluider, insbesondere wässriger Reaktionsmedien mittels einer kombinierten Anwendung einer Wärme- und einer UV-Behandlung durch Bestrahlung offenbart, dadurch gekennzeichnet, dass die Wärmebehandlung des Reaktionsmediums bei einer Sterilisationstemperatur von 40°C bis 135°C und die Bestrahlung bei einer Bestrahlungsdichte von 5 bis 300 W/m²erfolgt.

Es besteht somit Bedarf nach einer Methode zum Aufbringen von flexiblen, profilierten Hohlzylindern auf zylinderförmige Körper, die einfach auszuführen ist und die mechanische Belastung der Komponenten derart niedrig hält, dass eine Abnutzung und/oder gar Zerstörung der Komponenten ausgeschlossen werden kann.

Weiterhin besteht Bedarf an einem Verfahren zum Aufbringen von flexiblen, profilierten Hohlzylindern auf zylinderförmige Körper, das im industriellen Maßstab durchgeführt werden kann. Insbesondere die zumindest teilweise Automatisierung des Prozesses ist hier von enormer Wichtigkeit, um z.B. die in den Anmeldungen WO-A 02/38502, WO-A 02/38191, WO-A 07/096057 und EP-A 1464342 beschriebenen Bestrahlungsvorrichtungen in einem industriellen Maßstab kostengünstig und reproduzierbar herstellen zu können.

Ausgehend vom bekannten Stand der Technik stellt sich demnach die Aufgabe, ein Verfahren bereitzustellen, mit dem flexible, profilierte Hohlzylinder auf zylinderförmige Körper aufgebracht werden können, ohne dass eine Komponente hierbei einen Schaden nimmt. Das gesuchte Verfahren soll zumindest teilweise automatisierbar sein. Es soll einfach und kostengünstig auszuüben sein. Ferner stellt sich die Aufgabe, eine Vorrichtung bereitszustellen, mit dem das gesuchte Verfahren ausgeübt werden kann. Die gesuchte Vorrichtung soll intuitiv zu bedienen und ebenfalls kostengünstig sein.
Das gesuchte Verfahren soll die repoduzierbare Herstellung von Bestrahlungsvorrichtungen ermöglichen, die über wohldefinierte Kanalgeometrien verfügen und die damit wirkungsvolle und reproduzierbare Ergebnisse bei der Bestrahlung von fluiden Medien mittels elektromagnetischer Strahlung, zum Beispiel zum Zweck der Inaktivierung von Mirkoorganismen und/oder Viren mit UV-Strahlung, erzielen.
Überraschend wurde gefunden, dass sich flexible, profilierte Hohlzylinder einfach auf einen zylinderförmigen Körper aufbringen lassen, wenn die profilierten Hohlzylinder entlang der Längsachse gelängt (d.h. axial gestreckt) werden.

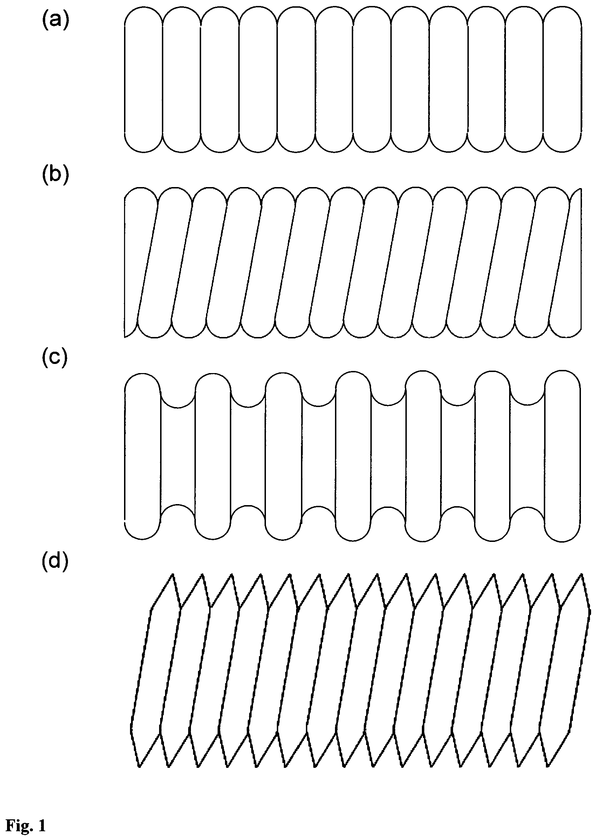

Gegenstand der vorliegenden Erfindung ist daher ein Verfahren gemäss Anspruch 1. Das erfindungsgemäße Verfahren ist für flexible, profilierte Hohlzylinder anwendbar, deren Profilierung zumindest teilweise in Form von Wellen oder Rillen besteht, die nicht in Richtung der Längsache verlaufen. Beispiele solcher flexibler, profilierter Hohlzylinder sind in Figur 1 schematisch dargestellt. Weitere Profilierungen sind denkbar.

Die Längung des Hohlzylinders bedeutet, dass der Hohlzylinder entlang seiner Längsachse gestreckt wird, so dass seine Länge zunimmt. Erfindungsgemäß beträgt der Grad der Längung von 1% bis 30%, bevorzugt von 5% bis 20%, besonders bevorzugt von 10 bis 15%, so dass die Länge des Hohlzylinders um den genannten Prozentsatz zunimmt. Überraschend wurde gefunden, dass durch eine erfindungsgemäße Längung der kleinste Innendurchmesser des Hohlzylinders, der durch die Windungen/Engstellen der Profilierung gebildet wird, zunimmt. Erfolgt eine Längung über einen Schwellenwert hinaus, so kann der kleinste Durchmesser wieder abnehmen. Der Schwellenwert ist dabei abhängig vom Material und der Profilierung. Der Schwellenwert kann einfach empirisch ermittelt werden. Weiterhin kann es bei zu großer Längung auch zu Schäden am profilierten Hohlzylinder kommen. Eine übermäßige Längung sollte daher vermieden werden. Um die Belastung des Materials des profilierten Hohlzylinders zu minimieren, sollte der Zeitraum der Längung minimiert werden. Die für den jeweiligen Fall optimale Dauer der Längung kann empirisch ermittelt werden. Parameter, welche die optimale Dauer einer Längung bestimmen sind z.B. Material, Dicke und Länge des Hohlzylinders, Temperatur, Durchsatz, und viele mehr. Bevorzugt erfolgt die erfindungsgemäße Längung in Schritt (a) nicht länger als zwei Minuten, wenn der gelängte Hohlzylinder aus PTFE (Polytetrafluorethylen) besteht und bei Raumtemperatur (15°C bis 25°C) verarbeitet wird.

Die erfindungsgemäße Längung des Hohlzylinders kann erfolgen, indem der Hohlzylinder an einem Ende fixiert und am anderen Ende gefasst und gestreckt wird. Z.B. kann der Hohlzylinder an einem Ende aufgehängt werden. Durch das eigene Gewicht des Hohlzylinders erfolgt bereits eine gewisse Längung. Durch Anbringen von Gewichten und/oder durch Ziehen am anderen Ende kann die Längung vergrößert werden. Der zylinderförmige Körper kann von oben oder unten in den gelängten Hohlzylinder eingebracht werden.

Bevorzugt erfolgt die Längung durch Fassen des Hohlzylinders an mindestens einer Stelle, bevorzugt an mindestens zwei Stellen. Bevorzugte Stellen zum Fassen des Hohlzylinders sind die beiden Enden. Bei einer Fassung an zwei Stellen, kann eine Fassungsstelle (die erste) festgehalten werden, während die andere Fassungsstelle von der ersten entfernt wird. Dadurch erfolgt eine Längung des Hohlzylinders. Ebenso ist es denkbar, dass sich beide Fassungsstellen voneinander entfernen.
In einer bevorzugten Ausführungsform des erfindungsgemäßen Verfahrens erfolgt die Fassung des Hohlzylindern an mehreren Stelle entlang des Hohlzylinders. Die Fassungsstellen sind bevorzugt gleichmäßig über die Länge des Hohlzylinders verteilt. Bevorzugt befinden sich an den beiden Enden des Hohlzylinders Fassungsstellen.
Die optimale Zahl der Fassungsstellen hängt von der Länge, Form und dem Material des Hohlzylinders ab und kann empirisch ermittelt werden. In einer bevorzugten Ausführungsform beträgt der Abstand zweier benachbarter Fassungstellen 300 mm bis 500 mm bei Verwendung eines PTFE-Wendelschlauches mit einem Durchmesser von 5 bis 20 cm als profiliertem Hohlzylinder.

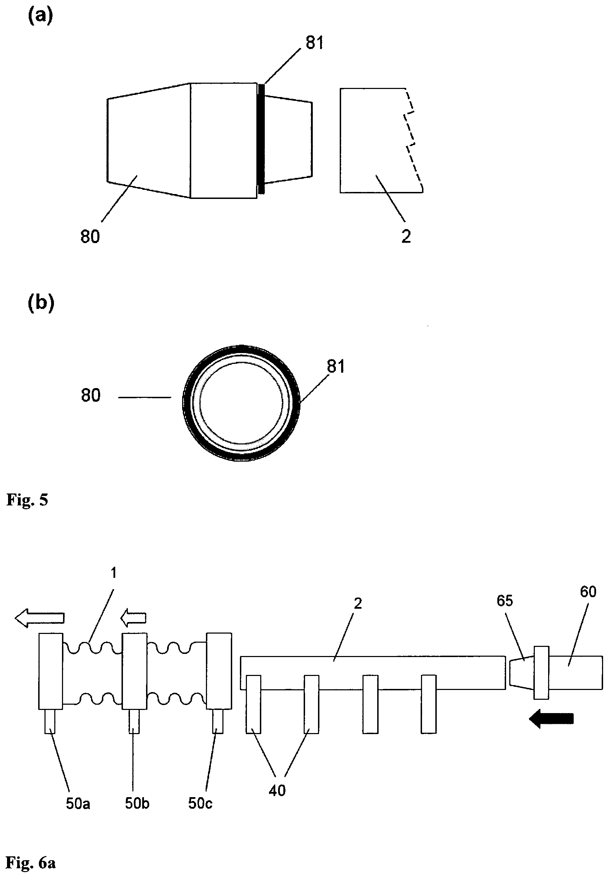

Beim Einbringen des zylinderförmigen Körpers in den gelängten Hohlzylinder in Schritt (b) des erfindungsgemäßen Verfahrens ist es unerheblich, ob der Körper in den Hohlzylinder geschoben oder der Hohlzylinder auf den Körper gezogen oder geschoben wird, oder ob eine Kombination der Bewegungen und Kräfte erfolgt. Eine entsprechende Vorrichtung sorgt dafür, dass Körper und Hohlzylinder in eine vorgegebene Position zueinander gelangen (Beispiel s.u.). Wenn im Folgenden davon die Rede ist, dass der Hohlzylinder auf den Körper gezogen oder geschoben oder der Körper in den Hohlzylinder geschoben oder gezogen wird, ist damit stets eine Kraft und eine resultierende relative Bewegung zwischen Hohlzylinder und Körper gemeint, die Hohlzylinder und Körper in eine vorgegebene Position zueinander bringt.
In einer bevorzugten Ausführungform erfolgt Schritt (b) unter Verwendung einer so genannten Zentrierungsvorrichtung oder Einführungshilfe. In einer bevorzugten Ausführungsform des erfindungsgemäßen Verfahren ist die Zentrierungsvorrichtung oder Einführungshilfe ein Aufsatz, der an dem Ende des zylinderförmigen Körpers angebracht wird, mit dem voran der Körper in den Hohlzylinder eingebracht wird. Der einzubringende Körper verfügt ggf. über scharfe Kanten, die beim Einbringen des Körpers in den Hohlzylinder zu Abschabungen und/oder Kratzern und/oder Schnitten an den Windungen/Engstellen der Profilierung des Hohlzylinders führen können. Der gelängte Hohlzylinder lässt sich nicht in allen Fällen über seine gesamte Länge entlang seiner Längsachse ausrichten. Bei einer horizontalen Lage des gelängten Hohlzylinders hängt der flexible Hohlzylinder zwischen zwei Fassungsstellen ggf. ein wenig herunter. Der einzubringende zylinderförmige Körper könnte beim Einführen gegen Windungen/Engstellen stoßen und Schaden anrichten. Dies wird durch Verwendung einer Zentrierungsvorrichtung oder Einführungshilfe wirksam verhindert, die bevorzugt konisch ausgeführt ist und den einzubringenden Körper führt. Ein Beispiel einer Zentrierungsvorrichtung oder Einführungshilfe ist in Figur 5 gegeben.

In Schritt (c) wird die Kraft zur Längung des Hohlzylinders aufgehoben, so dass der Hohlzylinder entspannt und seine Länge abnimmt. Der kleinste Durchmesser des Hohlzylinders nimmt durch diese Entspannung wieder ab und der Hohlzylinder umschließt den zylinderförmigen Körper.
Schritt (c) erfolgt dabei möglichst gleichmäßig über die gesamte Länge des Hohlzylinders, damit keine Spannungen innerhalb des profilierten Hohlzylinders entstehen. Der Hohlzylinder soll gleichmäßig und einheitlich auf den zylinderförmigen Körper aufgebracht werden. Dies geschieht besonders wirkungsvoll unter Verwendung so genannter Matritzen, welche die Entspannung führen und die Adjustierung zwischen Hohlzylinder und zylinderförmigem Körper erleichtern. In einer bevorzugten Ausführungform verfügen die Matritzen über ein Profil, das dem "Negativ" des Hohlzylinderprofils entspricht. Dadurch wird sichergestellt, dass eine maximale Kontaktfläche erzeugt wird und die Kräfte zur Längung und Adjustierung gleichmäßig in den profilierten Hohlzylinder eingebracht werden können.

Es sei darauf hingewiesen, dass das erfindungsgemäße Verfahren in abgewandelter Form auch zum Einbringen eines flexiblen, profilierten Hohlzylinders in einen anderen Hohlzylinder geeignet ist. Durch erfindungsgemäße Längung des profilierten Hohlzylinders nimmt nicht nur sein kleinster Innendurchmesser zu, sondern überraschenderweise auch sein größter Außendurchmesser ab. Soll ein flexibler, profilierter Hohlzylinder in einen Hohlzylinder eingebracht werden, dessen Innendurchmesser kleiner ist als der größte Außendurchmesser des profilierten Hohlzylinders, so kann durch erfindungsgemäße Längung das Einbringen einfach, mit geringem Kraftaufwand und bei Vermeidung von Schäden an den Hohlzylindern vollbracht werden. Die hier angestellten Überlegungen und Lösungen lassen sich somit auch auf dieses technische Problem anwenden.

Überraschend wurde gefunden, dass sich das erfindungsgemäße Verfahren zum Aufbringen flexibler, profilierter Hohlzylinder auf zylinderförmige Körper sehr gut zur Herstellung von Bestrahlungsvorrichtungen eignet. Unter einer Bestrahlungsvorrichtung wird eine Vorrichtung verstanden, die von einem fluiden Medium durchströmt wird, wobei das Medium einer elektromagnetischen Strahlung ausgesetzt wird. Bei Verwendung von UV-Strahlung lässt sich eine solche Bestrahlungsvorrichtung z.B. zur Reduktion von Mikroorganismen und/oder Viren in fluiden Medien einsetzen. Bei Verwendung von IR-Strahlung lässt sich eine solche Bestrahlungsvorrichtung z.B. zur Erwärmung eines fluiden Mediums einsetzen. Weitere Verwendungsmöglichkeiten sind denkbar, wie beispielsweise Reaktoren für photochemisch aktivierte und/oder photochemisch ablaufende Prozesse.

In einer bevorzugten Ausführungsform des erfindungsgemäßen Verfahrens wird als flexibler, profilierter Hohlzylinder ein Wendelschlauch eingesetzt. Der Wendelschlauch besteht bevorzugt aus einem Kunststoff, z.B. PTFE (Perfluorethylen). Als zylinderförmiger Körper wird bevorzugt ein für elektromagnetische Strahlung durchsichtiges Rohr verwendet. Bevorzugt wird ein Quarzglasrohr verwendet, das für UV-Strahlung durchlässig ist. Das erfindungsgemäße Verfahren eignet sich besonders zur Herstellung von solchen Bestrahlungsvorrichtungen - nachstehend auch Bestrahlungsmodule genannt, die dadurch gekennzeichnet sind, dass ein Wendelschlauch auf ein Stützrohr so aufgebracht ist, dass zwischen dem Wendelschlauch und dem Stützrohr eine Presspassung vorliegt. Unter Presspassung wird verstanden, dass der kleinste Innendurchmesser des Wendelschlauchs im kräftefreien Zustand kleiner ist als der Außendurchmesser des zylinderförmigen Körpers. Durch die erfindungsgemäße Längung wird der kleinste Innendurchmesser des Wendelschlauchs vergrößert und erreicht einen Wert, der größer als der Außendurchmesser des Stützrohres ist. Dadurch ist ein einfaches Einbringen des Stützrohres in den Wendelschlauch ohne übermäßige Kraftaufbringung und damit ohne Schädigung des Schlauchs und des Stützrohres möglich. Wird die Längung aufgehoben, nimmt der kleinste Innendurchmesser des Schlauches ab und die Windungen des Schlauches umschließen das Quarzglasrohr formschlüssig und gleichmäßig. Durch die Presspassung üben die Windungen einen Druck auf das Quarzglas aus, so dass der Schlauch nicht vom Quarzglasrohr abrutschen kann. Ferner kommt es bei der Verwendung der Bestrahlungsmodule zu keiner Kurzschlußströmung zwischen benachbarten Helikalwendeln. Es entsteht in reproduzierbarer Weise ein Kanal, der über seine Länge einen gleichförmigen Querschnitt aufweist und helikal um das Stützrohr verläuft.

Gegenstand der vorliegenden Erfindug ist somit auch ein Verfahren zur Herstellung von Bestrahlungsmodulen. Ebenso sind Bestrahlungsmodule, die mittels des erfindungsgemäßen Verfahrens hergestellt worden sind, Gegenstand der vorliegenden Erfindung. Die erfindungsgemäßen Bestrahlungsmodule lassen sich z.B. zur Bestrahlung von fluiden Medien zum Zwecke der Sterilisation und/oder Virusinaktivierung einsetzen.

Die Sterilisation oder Keimreduzierung in fluiden Medien ist in vielen Prozessen ein wichtiger Verfahrensschritt. Kontaminationen mit aktivem, das heißt vermehrungsfähigem, biologischem Material wie Mikroorganismen oder Viren stellt oft eine Gefährdung der Produktsicherheit dar, der wirkungsvoll begegnet werden muss.

Die Keimreduktion durch Inaktivierung mit ultravioletter Strahlung, besonders mit UV-C Strahlung und speziell bei etwa 254 nm Wellenlänge ist seit langem bekannt und wird vielfach praktisch angewandt. Beispiele sind neben Oberflächenentkeimungen auch die Behandlung flüssiger Medien wie Trink- und Abwässer. Eine wesentliche technische Herausforderung ist dann gegeben, wenn neben den zu inaktivierenden Mikroorganismen auch Wertstoffe enthalten sind, die zu einem gewissen Grad auch durch die Strahlung geschädigt werden können. Solche Anforderungen sind typisch für die Entkeimung im Bereich der Lebensmittel und von bio-pharmazeutischen Flüssigkeiten, wie beispielsweise Proteinlösungen. Zusätzliche Schwierigkeiten ergeben sich dann, wenn die Trübung der prozessierten Flüssigkeit im Bereich der UV-C Strahlung hoch und damit die Eindringtiefe der inaktivierenden Strahlung gering ist. Solche Anwendungen verlangen nach technischen Systemen, die trotz der hohen Trübung eine homogene Bestrahlung, das heißt eine enge Dosisverteilung, realisieren können. Bei durchströmten Apparaten ist hier zusätzlich eine gewisse Verweilzeit in der Bestrahlungszone, gleichbedeutend mit Bestrahlungszeit, vorzusehen. Die systemspezifische Verweilzeitverteilung führt dabei auch zu einer breiten, das heißt inhomogenen, Dosisverteilung in der Flüssigkeit.

Das erfindungsgemäße Bestrahlungsmodul für die Bestrahlung von Fluiden mit elektromagnetischer Strahlung umfasst ein UV-transparentes Rohr (zylinderförmiger Körper) zur Aufnahme einer zentral angeordneten, länglichen Strahlenquelle und einen auf das Rohr aufgebrachten Wendelschlauch (profilierter Hohlzylinder), so dass zwischen dem Wendenschlauch und Rohr je nach Profilierung ein oder mehrere wendelförmige Kanäle entstehen, in denen ein prozessiertes Fluid, während der Verweilzeit in der Bestrahlungszone, bestrahlt werden kann.

Ein wendelförmiger Kanal zwingt das durchströmende Fluid in eine helikale Strömung. Das besondere Kennzeichen des durchströmten Kanals ist eine intensive, über die gesamte Länge herrschende gleichförmige (durch sogenannte Dean-Wirbel hervorgerufene) Quervermischung senkrecht zur Hauptrichtung der Produktströmung - sogar bei laminaren Strömungsverhältnissen. Dadurch wird gewährleistet, dass die von der Strahlenquelle entfernteren Flüssigkeitsschichten, die besonders bei stark lichtabsorbierenden Medien keine oder wenig UV-Strahlung erhalten, einen intensiven Austausch mit den UV-bestrahlten Schichten nahe der Strahlenquelle eingehen.

Besonders bevorzugt hat ein solcher Kanal ein rechteckförmiges (vorzugsweise mit abgerundeten Ecken), trapezförmiges oder halbrundes Querschnittsprofil. Ein Kanal weist insbesondere eine Tiefe von 1 bis 100 mm, bevorzugt von 2 bis 50 mm, und eine mittlere Breite von 1 bis 200 mm, bevorzugt von 2 bis 50 mm, im Querschnittsprofil auf.

Ganz besonders bevorzugt ist eine Bauform des profilierten Hohlzylinders, in der die Profilierung in einem einzigen innen entlang der Mantelfläche wendelförmig laufenden Kanals besteht, der eine Steigung von 2 bis 20° (Steigungswinkel), bevorzugt von 4 bis 10°, aufweist.
Das Material des Rohres, durch das die Bestrahlung der Flüssigkeit erfolgt, sollte weitgehend strahlendurchlässig sein. Geeignete Materialien sind je nach Wellenlängenbereich der elektromagnetischen Strahlung beispielsweise Glas oder Kunststoffe. Das Material, das den Kanal ausbildet und nicht durchstrahlt wird, sollte insbesondere formstabil sein. Geeignete Werkstoffe sind beispielsweise metallische Werkstoffe, Kunststoffe, Keramiken, Glas oder Komposit-Materialien. Sollte dieses Material mindestens weitgehend für die verwendete elektromagnetische Strahlung transparent sein, so kann eine ergänzende oder auch alternative Bestrahlung auch durch dieses Bauteil von außen erfolgen. Eine weitere Ausführung kann eine spiegelnde Beschichung auf der Innenseite des profilierten Hohlzylinders sein, so dass es zu einer Spiegelung der Strahlung und damit zu einer Intensivierung der Strahlung in der Bestrahlungszone kommt.

In einer bevorzugten Ausführungform wird das erfindungsgemäße Bestrahlungsmodul durch Aufbringen eines Wendelschlauches aus PTFE auf ein für UV-Strahlen durchlässiges Stützrohr erzeugt, wobei der kleinste Innendurchmesser des Wendelschlauches, gegeben durch die inneren Windungen, geringfügig kleiner als der Außendurchmesser des Stützrohres ist. Unter geringfügig kleiner, wird verstanden, dass der kleinste Innendurchmesser des Wendelschlauches um 0,01% bis 5%, bevorzugt um 0,1% bis 3% kleiner ist als der Außendurchmesser des Stützrohres. Dadurch wird eine straffe, kraftschlüssige Verbindung zwischen beiden Elementen des Bestrahlungsmoduls hergestellt (Presspassung). Hierdurch lassen sich die sonst durch Spalte zwischen den Strömungskanälen verursachten axialen Kurzschlussströmungen verhindern, die eine starke Verbreiterung der Verweilzeitverteilung zur Folge hätten. Ein Aufblähen des Wendelschlauches infolge des bei größeren Produktströmen ansteigenden Druckverlustes ist wegen der Ausbildung von Kurzschlussströmungen unerwünscht und wird z.B. durch eine entsprechend dimensionierte Wandstärke des Wendelschlauches und/oder in den Wendelschlauch eingelegte Metallarmierungen und/oder eine Ummantelung verhindert. Die Gestaltung eines Mantelrohres (Ummantelung) ist dabei günstigerweise so festzulegen, dass der Innendurchmesser des Mantels geringfügig kleiner ist als der Schlauchaußendurchmesser, um ohne nennenswerte Schlauchdeformation einen zusätzlichen Anpressdruck zu erzeugen. Bei kleinen Druckverlusten kann ein auf den Wellschlauch einfach aufzubringender Schrumpfschlauch die Druckstabilität verbessern.

Der Strahlungseintrag erfolgt über eine zentral in dem Stützrohr eingebrachte längliche Strahlungsquelle oder von außen mit um den Wendelschlauch angeordneten Strahlungsquellen. Bevorzugt erfolgt eine Bestrahlung durch eine innen im Stützrohr angebrachte Strahlungsquelle. Das Stützrohr ist zur UV-C-Behandlung bevorzugt als für UV-C-Strahlen durchlässiges Quarzrohr ausgeführt. Da eine Reinigung der Kanäle aufgrund ihrer schlechten Zugänglichkeit schwierig ist, werden die erfindungsgemäßen Bestrahlungsmodule in einer bevorzugten Ausführungsform als steril oder keimarm verpackte Einwegelemente ausgeführt. Einwegsysteme sind besonders in der biopharmazeutischen Industrie verbreitet, da man sich durch den Einsatz von Einwegsystemen aufwändige Reinigungsvalidierungen spart. Die Anforderungen an solche Einwegsysteme sind, dass diese kostengünstig hergestellt werden können und eine reproduzierbare Qualität aufweisen. Die Qualität bezieht sich hierbei insbesondere auf gleichbleibende Dimensionierung und gleichbleibende Oberflächeneigenschaften des Stützrohres (keine Gleitmittel, kein Film, keine Kratzer). Die erfindungsgemäßen Bestrahlungsmodule erfüllen diese Anforderungen, weil das erfindungsgemäße Verfahren zur Herstellung der Bestrahlungsmodule reproduzierbare Ergebnisse liefert, automatisiertbar und damit in einem industriellen Maßstab ausführbar, sowie kostengünstig ist und ohne Gleitmittel auskommt.

Die erfindungsgemäßen Bestrahlungsmodule werden deshalb erfindungsgemäß als in einem Reinraum verpackte, zertifizierte, schnell und einfach austauschbare Einwegmodule für den Einsatz in GMP-kontrollierten biopharmazeutischen oder Lebensmittelprozessen zur Verwendung empfohlen (GMP=Good Manufacturing Practice).

Die erfindungsgemäßen Bestrahlungsmodule weisen weiterhin mindestens zwei Anschlüsse zum Einleiten und Ausführen eines Fluids in den helikalen Bestrahlungsraum auf. Beispiele, wie ein Wendelschlauch auf einem Stützkörper mit Anschlüssen versehen werden kann, finden sich in EP 1464342 A1.

Gegenstand der vorliegenden Erfindung ist ferner eine Vorrichtung zum Aufbringen von flexiblen, profilierten Hohlzylindern auf zylinderförmige Körper gemäss Anspruch 9. Die erfindungsgemäße Vorrichtung verfügt über Mittel zur Fassung eines flexiblen, profilierten Hohlzylinders an einer oder mehreren Stellen, über Mittel zur Längung des Hohlzylinders und über Mittel zum Einbringen des Körpers in den profilierten Hohlzylinder.
Die Fassung eines Hohlzylinders erfolgt bevorzugt an mindestens zwei Stellen. Die Mittel zur Fassung der Hohlzylinder (Fassungselemente) fassen einen Hohlzylinder bevorzugt an den Enden. In einer besonders bevorzugten Ausführungsform sind weitere Fassungselemente vorzugsweise gleichförmig über die Länge des Hohlzylinders verteilt. Alle Fassungselemente oder alle Fassungselemente bis auf eines sind beweglich ausgeführt, so dass sie nach Fassen des Hohlzylinders gegeneinander bewegt werden können und so der Hohlzylinder entlang seiner Längsachse gestreckt (gelängt) werden kann. Die Bewegung der Fassungselemente erfolgt z.B. durch Schrittmotoren oder Ähnliches.
In einer bevorzugten Ausführungsform verfügt die erfindungsgemäße Vorrichtung über Positionsgeber, welche die Position eines jeden Fassungselements feststellen lassen, so dass die Längung und/oder die Adjustierung und/oder die Entspannung geregelt verläuft.
Die Mittel zum Einbringen des zylinderförmigen Körpers in den profilierten Hohlzylinder bestehten z.B. aus einer motorgetriebenden Schubstange, welche den z.B. auf Stützelementen gelagerten Körper in den Hohlzylinder einbringt.

In einer bevorzugten Ausführungsform umfasst die erfindungsgemäße Vorrichtung weiterhin Mittel zur Temperierung, um konstante Prozessbedingungen zu gewährleisten. Ferner kann es vorteilhaft sein, den profilierten Hohlzylinder vor der Längung und/oder Entspannung zu erwärmen, um z.B. seine Elastizität zu erhöhen und damit Schädigungen infolge der Längung zu verhindern.

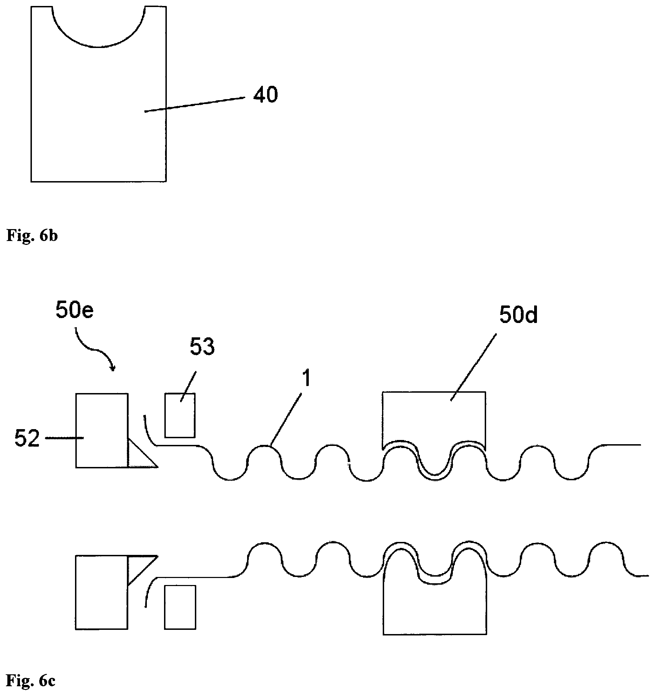

In einer bevorzugten Ausführungsform weist die erfindungsgemäße Vorrichtung weiterhin Mittel zur Adjustierung zwischen Hohlzylinder und zylinderförmigem Körper auf. Ein Beispiel ist in Figur 6c gezeigt.

In einer bevorzugten Ausführungsform weisen die Fassungselemente und/oder Mittel zur Adjustierung an der Kontaktstelle zum Hohlzylinder ebenfalls eine profilierte Kontur auf, die der Negativkontur des profilierten Hohlzylinders entspricht. Die Breite einer Aufnahme sollte mindestens eine, bevorzugt zwei bis zehn, besonders bevorzugt zwei bis fünf Profilwindungen betragen.

In einer bevorzugten Ausführungsform umfasst die erfindungsgemäße Vorrichtung weiterhin Mittel zur Erhöhung des Innendrucks innerhalb des profilierten Hohlzylinders, um den kleinsten Innendurchmesser des profilierten Hohlzylinders aufzuweiten. Die Druckerhöhung kann unterstützend zur Längung durchgeführt werden.

Weitere Merkmale der erfindungsgemäßen Vorrichtung sind Mittel zur Steuerung der Längung und/oder der Adjustierung des profilierten Hohlzylinders und/oder des Einbringens des zylinderförmigen Körpers in den gelängten Hohlzylinders.

Im folgenden werden Ausführungsbeispiele der Erfindung anhand von Figuren näher erläutert.

Es zeigen:
Figur 1 zeigt schematisch vier Beispiele für profilierte Hohlzylinder in der Seitenansicht, die unterschiedliche Profilierungen aufweisen: wendelförmige Windungen (a, c) sowie wellenförmige Windungen (b, d). Alle gezeigten Hohlzylinder weisen eine Profilierung auf, die in Form von Wellen oder Rillen besteht, die nicht in Richtung der Längsache verlaufen.

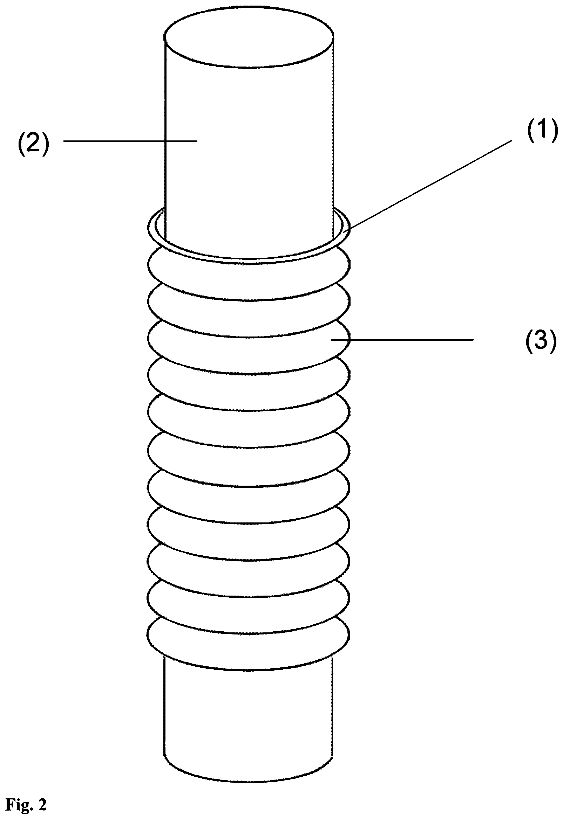

Figur 2 zeigt schematisch einen profilierten Hohlzylinder (1), der auf einen zylinderförmigen Körper (2) aufgebracht ist. Die Profilierung (3) besteht aus parallelen ringförmigen Windungen entlang der Mantelfläche des Hohlzylinders.

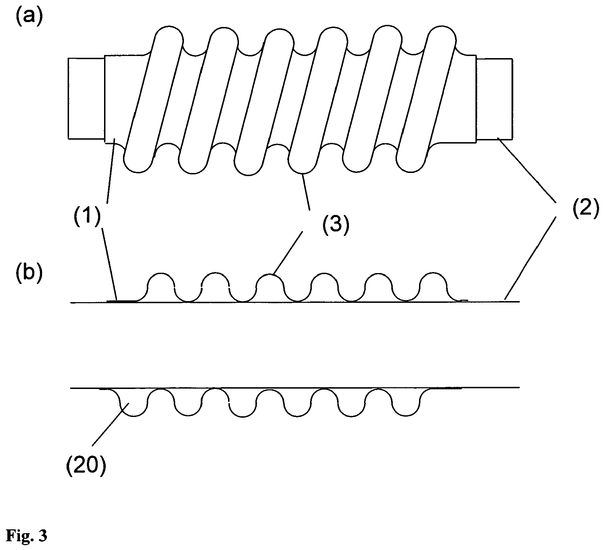

Figur 3 zeigt schematisch ein erfindungsgemäßes Bestrahlungsmodul, umfassend einen Wendelschlauch als profilierten Hohlzylinder (1) und ein zylinderförmiges Stützrohr (2): (a) in der Seitenaufsicht, (b) im Querschnitt von der Seite. Die wendelförmige Profilierung (3) führt in Kombination mit der Presspassung zu einem Kanal (20) der helikal um das Stützrohr verläuft. Spalte zwischen benachbarten Windungen des Kanals werden durch die Presspassung verhindert. In WO2007/096057 sind Beispiele beschrieben, wie der Kanal mit Anschlüssen zur Zu- und Abfuhr von fluidem Medien versehen werden kann.

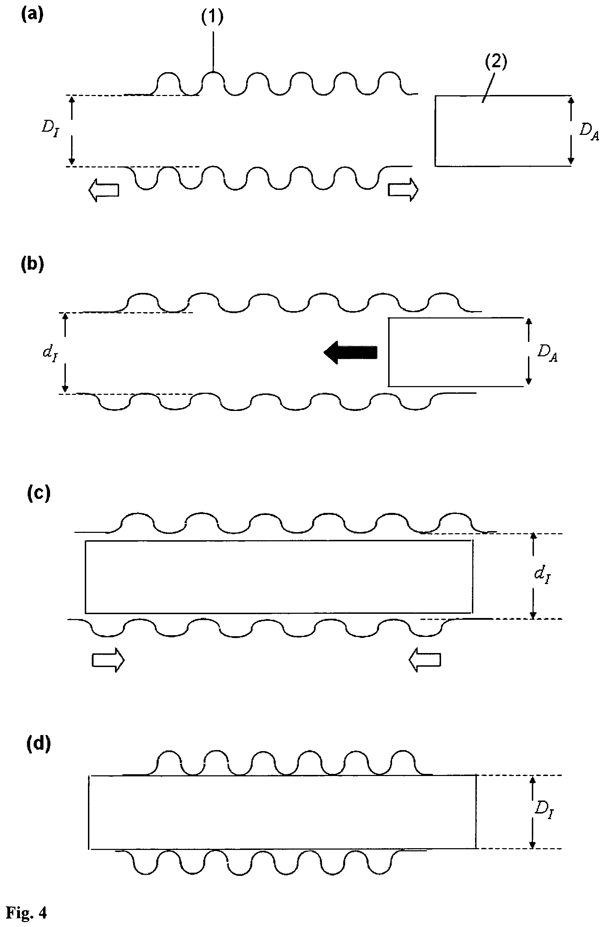

Figur 4 zeigt schematisch das erfindungsgemäße Verfahren zum Aufbringen elastischer, profilierter Hohlzylinder (1) auf einen zylinderförmigen Körper (2). In Fig. 4(a) wird der Hohlzylinder zunächst gelängt (angedeutet durch die weißen horizontalen Pfeile). Der kleinste Innendurchmesser *D_{I}* des Hohlzylinder ist im spannungsfreien Zustand bevorzugt geringfügig kleiner als oder genauso groß wie der Außendurchmesser *D_{A}* des zylinderförmigen Körpers. Durch die Längung steigt der kleinste Innendurchmesser auf einen Wert *d_{I}* an, der größer als der Außendurchmesser *D_{A}* ist (Fig. 4b).
   In Fig. 4(b) ist gezeigt, wie der zylinderförmige Körper einfach in den gelängten Hohlzylinder eingebracht werden kann (angedeutet durch den dicken schwarzen Pfeil).
   In Fig. 4(c) wird der Hohlzylinder entspannt (angedeutet durch die weißen Pfeile) und schließt sich in Fig. 4(d) formschlüssig um den zylinderförmigen Körper. Der Vorgang des Aufbringens sollte zur Schonung des Materials des profilierten Hohlzylinders, vor allem bei Verwendung von PTFE, in wenigen Minuten vorzugsweise deutlich unter einer Minute abgeschlossen sein, um bleibende Verformungen des Materials und damit eine Verringerung der Presspassung zu vermeiden.
Fig. 5 zeigt schematisch ein Beispiel einer Zentrierungsvorrichtung oder Einführungshilfe, um das Einbringen des zylinderförmigen Körpers in den gelängten Hohlzylinder zu vereinfachen und Schäden zu verhindern. Im vorliegenden Beispiel ist der zylinderförmige Körper (2) ein Glasrohr. Auf dieses Glasrohr wird die Zentrierungsvorrichtung (80) aufgesteckt. Ein Gummiring (81) sorgt dafür, dass das Glasrohr nicht beschädigt wird. Die konische Spitze der Zentrierungsvorrichtung erleichtert das Einbringen. Die Zentrierungsvorrichtung ist in Fig. 5(a) von der Seite und in Fig. 5(b) von der dem Glasrohr zugewandten Seite gezeigt.
Fig. 6a zeigt schematisch ein Beispiel einer erfindungsgemäßen Vorrichtung zum Aufbringen eines profilierten Hohlzylinders (1) auf einen zylinderförmigen Körper (2) von der Seite. Der zylinderförmige Körper ist im vorliegenden Beispiel als Rohr ausgeführt. Die Vorrichtung umfasst vier Stützen (40) zur Halterung und Führung des Rohres, eine Schubstange (60) mit konischer Spitze (65) zum Einschieben des Rohres in den Hohlzylinder und drei Fassungselemente (50a, 50b, 50c). Im vorliegenden Beispiel wird das Fassungselement (50c) festgehalten, während die Fassungselemente (50a) und (50b) beweglich sind und durch eine Antriebseinheit vom Fassungselement (50c) unter Streckung des Hohlzylinders wegbewegt werden. Damit die Streckung über die gesamte Länge des Hohlzylinders gleichmäßig erfolgt, muss sich das Fassungselement (50a) doppelt so schnell vom Element (50c) fortbewegen, wie Fassungselement (50b). Bei Verwendung von Schrittmotoren muss das Fassungselement (50a) bei jedem Schritt, den sich Fassungselement (50b) vom Element (50c) fortbewegt, zwei Schritte in dieselbe Richtung vornehmen. Dies wird durch die unterschiedlich langen weißen Pfeile oberhalb der Fassungselemente angedeutet. Analoges gilt für die Entspannung.
Fig. 6b zeigt schematisch eine Stütze (40) aus Fig. 6a von vorne.
Fig. 6c zeigt schematisch und beispielhaft zwei verschiedene Typen von Fassungselementen (50d, 50e) im Querschnitt. Fassungselement (50e) dient der Fassung eines profilierten Hohlzylinders (1) an einem seiner Enden. Es umfasst einen Ring (52) mit konischer Führung zum Eingreifen in den Hohlzylinder und einen weiteren Ring (53). Die Wulst am Ende des profilierten Hohlzylinders wird zwischen die Ringe (52) und (53) z.B. mittels einer Schraubverbindung (hier nicht gezeigt) geklemmt.
   Das Fassungselement (50d) dient der Fassung eines profilierten Hohlzylinders zwischen seinen Enden. Es weist eine Kontur auf, die dem Negativprofil des Hohlzylinders entspricht. Die Kontur dient der Aufnahme und Fixierung des Hohlzylinders und ermöglicht daneben sowohl die Adjustierung zwischen Hohlzylinder und eingebrachtem Körper als auch die gleichmäßige Entspannung des gelängten Hohlzylinders.

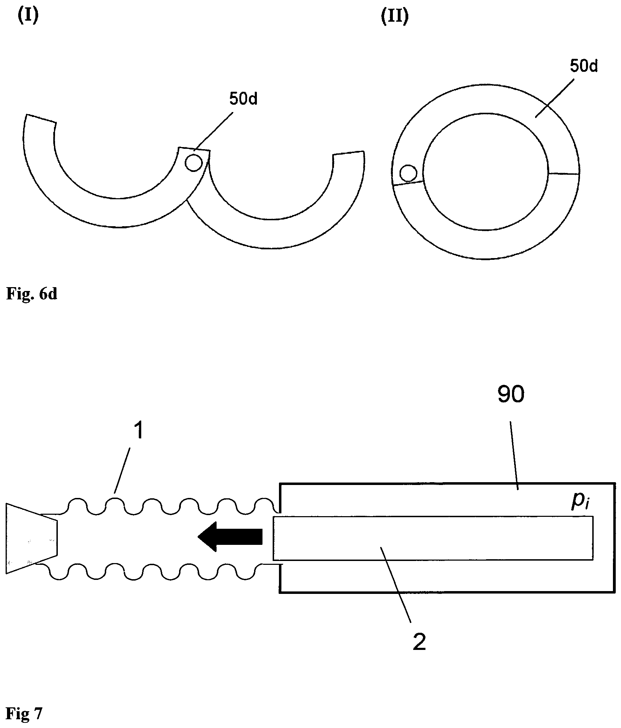

Fig. 6d zeigt schematisch das Fassungselement (50d) von vorne. Es wird in der obigen Beschreibung auch als Matrize bezeichnet. Es besteht aus zwei halbkreisförmigen Elementen, die über ein Schanier miteinander verbunden sind. In
Fig. 6d (I) ist das Element geöffnet und kann einen Hohlzylinder aufnehmen. In Fig. 6d (II) ist das Element geschlossen. Die offenen Enden der halbkreisförmigen Elemente könne z.B. durch Schraubverbindungen miteinander verbunden werden (hier nicht gezeigt), um ein ungewolltes Öffnen und/oder Herausrutschen des gefassten Hohlzylinders zu verhindern.
Fig. 7 zeigt schematisch, wie der Innenraum des profilierten Hohlzylinders unter einen erhöhten Druck *p*ᵢ gesetzt werden kann, so dass das Einschieben des Rohres zusätzlich erleichtert wird. Diese Ausführung kann sowohl alleine ein ausreichendes Aufweiten des Innendurchmessers des profilierten Hohlzylinders hervorrufen oder in Kombination mit einer Längung des profilierten Hohlzylinders ausgeführt werden.

### Bezugszeichen

- 1: profilierter Hohlzylinder
- 2: zylinderförmiger Körper
- 3: Profilierung
- 20: wendelförmiger Kanal
- 40: Stützen
- 50a: Fassungselement, fixiert
- 50b: Fassungselement, beweglich
- 50c: Fassungslement, beweglich
- 50d: Fassungselement für Mitte = Matrize
- 50e: Fassungselement für Ende
- 52: Ring mit konischer Führung
- 53: Ring
- 60: Schubstange
- 65: konische Spitze
- 80: Zentrierungsvorrichtung / Einführungshilfe
- 81: Gummiring
- 90: Druckkammer

## Patentansprüche

1. Verfahren zum Aufbringen eines flexiblen, profilierten Hohlzylinders (1) auf einen zylinderförmigen Körper (2), mindestens umfassend die folgenden zeitlich aufeinander folgenden Schritte:
(a) Längen des Hohlzylinders (1) durch axiales Strecken um 1% bis 30% entlang seiner Längsachse derart, dass der kleinste Innendurchmesser des Hohlzylinders (1) zunimmt,
(b) Einbringen des zylinderförmigen Körpers (2) in den so gelängten Hohlzylinder (1), und abschließendes
(c) Entspannen des Hohlzylinders (1).

2. Verfahren nach Anspruch 1, **dadurch gekennzeichnet, dass** die Länge des profilierten Hohlzylinder durch die Längung um 1% bis 20% zunimmt.

3. Verfahren nach einem der Ansprüche 1 oder 2, **dadurch gekennzeichnet, dass** die Längung durch Fassen des Hohlzylinders an mindestens 2 Stellen erfolgt und sich die Fassungsstellen relativ voneinander fortbewegen.

4. Verfahren nach einem der Ansprüche 1 bis 3, **dadurch gekennzeichnet, dass** zum Einbringen des zylinderförmigen Körpers eine Zentrierungsvorrichtung (80) verwendet wird.

5. Verfahren nach einem der Ansprüche 1 bis 4, **dadurch gekennzeichnet, dass** vor und/oder während des Entspannens eine Adjustierung zwischen Hohlzylinder und Körper zur Verwirklichung einer gleichmäßigen Presspassung und Kanalgeometrie erfolgt.

6. Verfahren nach Anspruch 5, **dadurch gekennzeichnet, dass** die Adjustierung unter Zurhilfenahme von Matrizen (50d) stattfindet, die über eine Kontur verfügen, die der Negativprofilierung des Hohlzylinders entspricht.

7. Verfahren zur Herstellung von Bestrahlungsmodulen, **dadurch gekennzeichnet, dass** ein Wendelschlauch (1) auf ein Stützrohr (2) mittels eines Verfahren gemäß einem der Ansprüche 1 bis 6 aufgebracht wird.

8. Verfahren nach Anspruch 7, **dadurch gekennzeichnet, dass** der kleinste Innendurchmesser des Wendeischlauchs 1% bis 5% kleiner als der Außendurchmesser des Stützrohres ist.

9. Vorrichtung speziell ausgestaltet zum Aufbringen eines flexiblen, profilierten Hohlzylinders auf einen zylinderförmigen Körper mittels eines Verfahrens gemäss einem der vorhergehenden Ansprüche, mindestens umfassend Mittel zur Fassung des Hohlzylinders an einer oder mehreren Stellen, Mittel zur Längung des Hohlzylinders entlang seiner Längsachse und Mittel zum Einbringen des Körpers in den gelängten Hohlzylinder.

10. Vorrichtung nach Anspruch 9, **dadurch gekennzeichnet, dass** die Mittel zur Fassung mindestens zwei Fassungselemente (50a,50c) umfassen, die den Hohlzylinder an beiden Enden aufnehmen und von denen mindestens ein Fassungselement beweglich ausgeführt ist, so dass die Fassungselemente unter Längung des Hohlzylinders relativ voneinander entfernt werden können.

11. Vorrichtung nach einem der Ansprüche 9 oder 10, **dadurch gekennzeichnet, dass** die Mittel zur Fassung des Hohlzylinders mehr als zwei Fassungselemente umfassen, die gleichmäßig über die Länge des Hohlzylinders verteilt sind und die sich relativ voneinander entfernen können.

12. Vorrichtung nach einem der Ansprüche 9 bis 11, weiterhin umfassend Mittel zur Adjustierung des gelängten Hohlzylinders in Bezug zum zylinderförmigen Körper.

13. Vorrichtung nach einem der Ansprüche 9 bis 12, weiterhin umfassend Mittel zur Temperierung des Hohlyzlinders.

## Claims

1. Method of mounting a flexible, profiled hollow cylinder (1) onto a cylindrical element (2), which comprises at least the following temporally sequential steps:
(a) elongating the hollow cylinder (1) by axial stretching by 1% to 30% along its longitudinal axis such that the smallest inner diameter of the hollow cylinder (1) increases,
(b) inserting the cylindrical element (2) into the hollow cylinder (1) elongated in this way, and finally,
(c) relaxing the hollow cylinder (1).

2. Method according to Claim 1, **characterized in that** the length of the profiled hollow cylinder increases by 1% to 20% as a result of the elongation.

3. Method according to one of Claims 1 or 2, **characterized in that** the elongation is carried out by gripping the hollow cylinder at at least 2 points and the gripping points move relatively away from each other.

4. Method according to one of Claims 1 to 3, **characterized in that** a centering device (80) is used for inserting the cylindrical element.

5. Method according to one of Claims 1 to 4, **characterized in that**, before and/or during the relaxation, adjustment is carried out between the hollow cylinder and the element in order to produce a uniform press fit and channel geometry.

6. Method according to Claim 5, **characterized in that** the adjustment is carried out with the aid of matrices (50d) which have a contour which corresponds to the negative profiled contour of the hollow cylinder.

7. Method of producing irradiation modules, **characterized in that** a spiral tube (1) is mounted onto a supporting tube (2) using a method according to one of Claims 1 to 6.

8. Method according to Claim 7, **characterized in that** the smallest inner diameter of the spiral tube is 1% to 5% smaller than the outer diameter of the supporting tube.

9. Device specifically designed for mounting a flexible, profiled hollow cylinder onto a cylindrical element by means of a method according to one of the preceding claims, comprising at least means for gripping the hollow cylinder at one or more points, means for elongating the hollow cylinder along its longitudinal axis and means for inserting the element into the elongated hollow cylinder.

10. Device according to Claim 9, **characterized in that** the gripping means comprise at least two gripping elements (50a, 50c) which hold the hollow cylinder at both ends and of which at least one gripping element is movable, so that the gripping elements can be moved relatively away from each other while elongating the hollow cylinder.

11. Device according to one of Claims 9 or 10, **characterized in that** the means for gripping the hollow cylinder consist of more than two gripping elements which are distributed uniformly over the length of the hollow cylinder and can move relatively away from each other.

12. Device according to one of Claims 9 to 11 which also includes means for adjusting the elongated hollow cylinder in relation to the cylindrical element.

13. Device according to one of Claims 9 to 12 which also includes temperature control means for the hollow cylinder.

## Revendications

1. Procédé pour appliquer un cylindre creux souple et profilé (1) sur un corps cylindrique (2), le procédé comportant au moins les étapes ci-dessous, exécutées dans l'ordre indiqué :
(a) allongement du cylindre creux (1) par étirage axial de 1 % à 30 % le long de son axe longitudinal de manière à augmenter le plus petit diamètre intérieur du cylindre creux (1),
(b) insertion du corps cylindrique (2) dans le cylindre creux (1) ainsi allongé et ensuite
(c) relâchement du cylindre creux (1).

2. Procédé selon la revendication 1, **caractérisé en ce que** la longueur du cylindre creux profilé augmente de 1 % à 20 % lors de son allongement.

3. Procédé selon l'une des revendications 1 ou 2, **caractérisé en ce que** l'allongement s'effectue par saisie du cylindre creux en au moins deux emplacements, les emplacements de saisie s'écartant l'un de l'autre.

4. Procédé selon l'une des revendications 1 à 3, **caractérisé en ce que** pour insérer le corps cylindrique, on utilise un ensemble de centrage (80).

5. Procédé selon l'une des revendications 1 à 4, **caractérisé en ce qu'**avant et/ou pendant le relâchement s'effectue un ajustement entre le cylindre creux et le corps, de manière à réaliser un ajustement serré uniforme et une géométrie uniforme du canal.

6. Procédé selon la revendication 5, **caractérisé en ce que** l'ajustement a lieu en recourant à des matrices (50d) dont le contour correspond au profil négatif du cylindre creux.

7. Procédé de fabrication de modules d'irradiation, **caractérisé en ce qu'**un tuyau flexible spiralé (1) est appliqué sur un tube de soutien (2) au moyen d'un procédé selon l'une des revendications 1 à 6.

8. Procédé selon la revendication 7, **caractérisé en ce que** le plus petit diamètre intérieur du tuyau flexible spiralé est de 1 % à 5 % plus petit que le diamètre extérieur du tube de soutien.

9. Ensemble configuré spécialement en vue d'appliquer un cylindre flexible profilé creux sur un corps cylindrique au moyen d'un procédé selon l'une des revendications précédentes, l'ensemble comprenant au moins des moyens de saisie du cylindre creux en un ou plusieurs emplacements, des moyens d'allongement du cylindre creux le long de son axe longitudinal et des moyens d'insertion du corps dans le cylindre creux allongé.

10. Ensemble selon la revendication 9, **caractérisé en ce que** les moyens de saisie comprennent au moins deux éléments de saisie (50a, 50c) qui reprennent le cylindre creux à ses deux extrémités et dont au moins l'un est configuré de manière à pouvoir être déplacé de telle sorte que les éléments de saisie puissent être éloignés l'un de l'autre pour allonger le cylindre creux.

11. Ensemble selon l'une des revendications 9 ou 10, **caractérisé en ce que** les moyens de saisie du cylindre creux comprennent plus de deux éléments de saisie répartis régulièrement sur la longueur du cylindre creux et aptes à s'éloigner les uns des autres.

12. Ensemble selon l'une des revendications 1 à 11, comprenant en outre des moyens d'ajustement du cylindre creux allongé par rapport au corps cylindrique.

13. Ensemble selon l'une des revendications 9 à 12, comprenant en outre des moyens permettant d'amener et de maintenir le cylindre creux à une température contrôlée.
